# EUROPEAN PATENT APPLICATION

(11) **EP 0 788 771 A1**
(43) Date of publication of application: **13.08.1997**
(21) Application number: 96201695.2
(22) Date of filing: 18.06.1996
(51) Int. Cl.: A61B 17/08

(54) **Skin closure device**

(30) Priority: 26.10.1995 IE 950842
(71) Applicant: Boyle, Francis, Balinfull, County Sligo (IE)
(72) Inventor: Boyle, Francis, Balinfull, County Sligo (IE)
(74) Representative: Boyce, Conor

(57) **Abstract**

A skin closure device comprises a strip of material (10) having an adhesive (11) on one side for securing the strip (10) along one edge of a wound (15) and one or more threads (12) extending from one edge (13) of the strip (10) for tying to like threads (12) extending from the other edge of the wound (15). A second embodiment has two strips (10) connected by a common set of threads (12').

## Description

This invention relates to a skin closure device.

Conventionally, wounds and cuts are closed by stitching. However, this leaves needle scars. Also, anaesthetic is often required. Further, stitching does not permit ready readjustment of the wound later.

Accordingly, the present invention provides a skin closure device comprising a strip of material having an adhesive on one side for securing the strip along one edge of a wound and one or more threads extending from one edge of the strip.

In one embodiment the thread or threads have free ends for tying to a like thread or threads extending from a like strip adhesively secured along the other edge of the wound. Alternatively the free ends of the threads may be tied to a suture stitched into the skin along the other edge of the wound.

In another embodiment the device includes a further strip of material having an adhesive on one side for securing the strip along the other edge of the wound, the one or more threads being common to both strips and joining their adjacent edges.

Embodiments of the invention will now be described, by way of example, with reference to the accompanying drawings, in which:
Figure 1 is a plan view of a first embodiment of skin closure device according to the invention,
Figure 2 is a schematic cross-sectional view of the device of figure 1,
Figure 3 shows how two such devices are tied together to close a wound, and
Figure 4 is a plan view of a second embodiment of the invention.

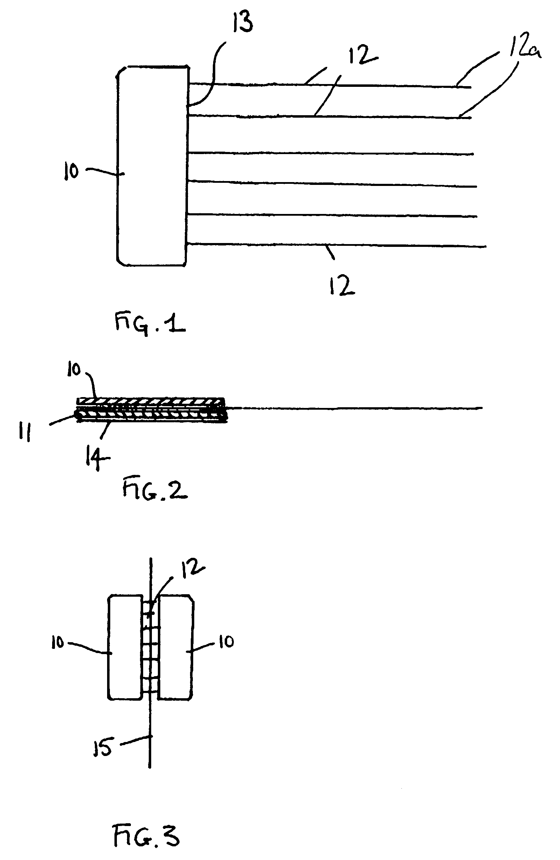

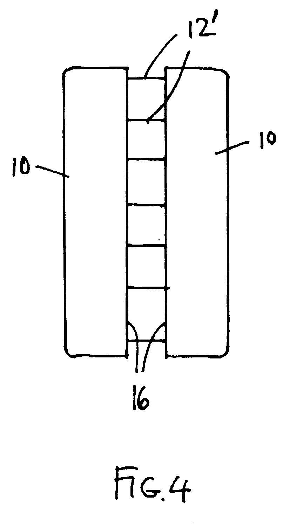

The first embodiment, figures 1 and 2, comprises a strip of flexible material 10 having an adhesive layer 11 on one side for securing the strip 10 to the skin along one edge of a wound. A plurality of threads 12 extending from one longitudinal edge 13 of the strip 10 have free ends 12a for tying to like threads extending from a like strip adhesively secured along the other edge of the wound. The other ends of the threads 12 are laminated between the adhesive layer 11 and the strip 10. A release paper 14 is provided on the adhesive layer 11. The device may be manufactured in a variety of sizes with different numbers of threads 12.

In use, figure 3, a pair of strips 10 is selected according to the size of wound 15 to be closed, the release paper 13 is removed from each, and they are stuck along respective edges of the wound with their edges 13 opposed across the wound so that the threads 12 extend towards one another. The threads 12 are then tied together in pairs across the wound 15 to close it.

If desired, according to the shape and size of the wound, the threads 12 may be tied together other than one-to-one in pairs. For example, a thread on one strip 10 may be tied to two or more threads on the other strip, and certain threads may not be used (they will then preferably be cut off). It is also possible to use strips 10 of different sizes and numbers of threads on each side of the wound respectively.

Advantages of this arrangement are that there are no needle scars and an anaesthetic is often not necessary. Also, the arrangement permits the opposite sides of the wound to be drawn together to a different extent at each point along the length of the wound, by appropriately adjusting the lengths of the tied threads extending between the adjacent edges of the strips. This ability, together with the ability to use different sized strips 10 on each side of the wound and to select which threads to tie, provides great flexibility in adapting to different wound shapes. In all cases the threads can be untied to re-adjust the wound as necessary.

As an alternative (not shown) to using a pair of strips 10 to close a wound as shown in Fig. 3, it is possible to use a single such strip on one side of the wound in conjunction with suture stitched into the skin on the opposite side of a wound. In that case the threads 12 would be tied to the suture. Again, adaptability to different wound sizes is provided and the threads can be untied to re-adjust the wound if necessary.

In a second embodiment of the invention (Figure 4), the device comprises two strips 10 as described above with a common set of threads 12' extending between their adjacent edges. Again the device may be made in a variety of sizes with different numbers and lengths of threads 12'. In use a device of suitable size and number and length of threads 12' is chosen and the strips 10 are located on opposite sides of the wound a suitable distance apart to ensure closure of the wound.

## Claims

1. A skin closure device comprising a strip of material (10) having an adhesive (11) on one side for securing the strip along one edge of a wound (15) and one or more threads (12 or 12') extending from one edge of the strip.

2. A skin closure device as claimed in claim 1, wherein the thread or threads (12) have free ends (12a) for tying to a like thread or threads extending from a like strip adhesively secured along the other edge of the wound.

3. A skin closure device as claimed in claim 1, comprising a further strip of material (10) having an adhesive (11) on one side for securing the strip along the other edge of the wound (15), the one or more threads (12') being common to both strips and joining their adjacent edges (16).
